Europäisches Patentamt

European Patent Office

Office européen des brevets

(11) Publication number: **0 386 680**
**A1**

## (12) EUROPEAN PATENT APPLICATION

(21) Application number: 90104214.3

(22) Date of filing: 05.03.90

(51) Int. Cl.⁵ **A61K 7/00, A61K 7/42**

(30) Priority: 07.03.89 US 320012

(43) Date of publication of application:
**12.09.90 Bulletin 90/37**

(84) Designated Contracting States:
**GR**

(71) Applicant: **PLOUGH, INC.**
**3030 Jackson Avenue**
**Memphis, TN 38151(US)**

(72) Inventor: **Agin, Patricia P.**
**2388 Cherry Spring Cove**
**Cordova, Tennesse 38018(US)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

(54) **Liposome compositions.**

(57) Sunless tanning of skin is accomplished by applying a topical composition containing a liposome-melanin complex.

EP 0 386 680 A1

## LIPOSOME COMPOSITIONS

### BACKGROUND

This invention relates to a complex of a liposome and entrapped melanin polymer compositions, compositions containing the complexes and methods for using the complexes as topical tanning agents for skin.

Melanin is the dark macromolecular pigment of skin which, in persons of light skin, is formed in the skin upon continued exposure to sunlight or ultraviolet radiation to form a brownish color. Such exposure to the sun or ultraviolet radiation causes darkening of preformed melanin (Meirowsky phenomenon), accelerated formation of new melanin, and retention of melanin in the epidermis as a result of thickening of the skin and transferral of the melanins to the keratinocytes. Melanins are naturally occurring and synthetic polymers containing phenolic hydroxyl, carboxyl, amine groups, and stable free radicals of the semiquinone type. Melanin can exist either in an oxidized quinoid form or reduced hydroquinoic form. Synthetic melanins are black heterogeneous polymers formed by autoxidation of catechol or of DOPA (3,4-dihydroxyphenylalanine) and are described as "catechol melanin" and "DOPA melanin" respectively. Although all natural and synthetic melanins are contemplated for use in this invention, DOPA melanins are preferred. The preparation of synthetic melanins is disclosed in Froncisz et al., Archives of Biochemistry and Biophysics, 202 No. 1, 289-303 (1980) and Horak et al, Molecular Pharmacology, 7, 429-433 (1971). Synthetic melanin is also available from Sigma Chemical Company, St. Louis, Missouri, U.S.A.

Enzyme Therapy in Lysosomal Storage Diseases, ed. Tager et al., pages 149-162, (1974) North-Holland Publishing Company discusses the possible use of liposomes as carriers for enzymes, and state on page 150:

"Liposomes --- consist of a series of concentric bilayers of lipids alternating with aqueous compartments, within which soluble material, such as enzymes, may be entrapped. The lipid composition may be varied considerably and thus gives liposomes of varying 'membrane' structure as well as differences in net charge."

Liposomes have also been used as carriers for proteins by Gregoriadis et al., Eur. J. Biochem. 24. 485-91 (1972).

Handjani-Vila, et al., International Journal of Cosmetic Science, 1 303-314 (1979), discuss the use of colorless tartaric aldehyde and dihydroxyacetone in liposomes, (page 309). However, the aldehyde and ketone require color development by reaction with the skin whereas applicant's compositions are colored and impart color to the skin.

Bishop, The Wall Street Journal, page 1 (Southwest Edition) August 26, 1988, discusses using genetically engineered melanin in microscopic plastic sponges for coating skin to block the suns rays. The article indicates that the lotion is not clear and may actually color the skin. In addition, the article indicates that the melanin sponges are too big to be absorbed by the skin. It is clear from the article that the "sponges" are not liposomes. Additionally, the microsponge product differs from the invention because the inventive product involved is absorbed by the stratum corneum and partially into the epidermis.

### SUMMARY

This invention relates to a means to tan skin without sunlight and protect skin against sunburn and actinic keratosis caused by the harmful rays of the sun by applying to the skin a topical composition containing from about $1 \times 10^{-6}$ mg/mL to about $5 \times 10^{-3}$ g/mL of melanin, wherein the melanin, either alone or in a composition containing color modifiers and other conventional additives, is entrapped in a liposome. The invention also relates to topical compositions comprising a liposome-melanin complex containing sufficient melanin to provide from about $1 \times 10^{-6}$ mg/mL to about $5 \times 10^{-3}$ g/mL based on the composition, and a topical pharmaeutical or cosmetic base.

The preferred concentrations of melanin in the compositions of this invention are from 0.5 to 5 mg/mL.

The color of the topical compositions of this invention can be altered to individual requirements by including in the compositions one or more colored materials which function as color modifying agents, e.g. beta-carotene, canthaxanthin, and riboflavin. The concentration of such agents depends on the specific final color sought. For example, if a golden tan color is required, a mixture of 0.0002% by weight riboflavin and 2% by weight canthaxanthin is added to the melanin composition. For a darker brown color, 0.5% by weight canthaxanthin and 0.0001% by weight beta-carotene can be added to the melanin composition.

This invention therefore includes topical compositions for tanning skin comprising a liposome-melanin complex containing sufficient melanin to provide from about $1 \times 10^{-6}$ mg/mL to about $5 \times 10^{-3}$ g/mL melanin, about $2 \times 10^{-2}$ g/mL of a color modifying agent or agents such as canthaxanthin, riboflavin, beta-carotene or mixtures thereof, and a pharmaceutical or cosmetic carrier.

It is believed that the compositions of this invention are non-toxic when the compositions' components are used in the stated concentration ranges.

## DETAILED DESCRIPTION

The liposome-melanin complex compositions of this invention are advantageous because they protect the skin from harmful UVA and UVB rays and at the same time provide a suntan color to the skin. The color agents in the compositons, i.e. melanin and color modifiers, penetrate into the stratum corneum and to a small extent, penetrate into the upper layers of the epidermis. Melanin, when applied alone, does not penetrate into the stratum corneum. The composition of this invention readily washes off with soap or detergent and water, leaving a very light residual color.

"Melanin", as used herein includes all melanins derived from natural sources and synthetic melanin such as DOPA-melanin available from Sigma Chemical Co., St. Louis, Missouri, under catalog number M-8631. Melanins prepared from catechol (2-hydroxy phenol) and melanin isolated or derived from natural sources are also suitable for use in this invention. The preferred melanin is DOPA-melanin.

"Liposome" as used herein includes pharmaceutically and/or cosmetically acceptable liposomes suitable for topical administration. Liposomes are a series of concentric bilayers of lipids alternating with aqueous compartments into which soluble or dispersible materials such as proteins, enzymes, corticosteroids, dyes, and other pharmaceuticals and cosmetics can be entrapped.

Among the liposomes useful in this invention are stratum corneum lipid liposomes formed from epidermal ceramides, cholesterol, palmitic acid and cholesterol sulfate as described in Abraham et al., The Journal of Investigative Dermatology, 90, 259-262 (1988).

Many lipids are suitable for use in making liposomes which can be used in this invention, many of which are commercially available, e.g. Liposome Kit is available from Sigma Chemical Company, St. Louis, Missouri under catalog number L4262. Liposome Kit L4262 contains L-alpha-phosphatidylcholine (egg yolk), dicetyl phosphate and cholesterol. It is a negatively charged lipsome mixture, another suitable negatively charged liposome mixture available from Sigma Chemical Company is L-4012 which contains L-alpha-phosphatidylcholine, dicetyl phosphate and cholesterol. Suitable positively charged liposome mixtures available from Sigma Chemical Company contains L-alpha-phosphatidylcholine, stearylamine and cholesterol (l-4137 and L-3887).

Categories of lipids in suitable liposomes are phospholipids, glycosphingolipids, ceramides, cholesterol sulfate and neutral lipids. Various combinations of these lipids are found in neonatal mouse, pig and human stratum granulosum and stratum corneum. Other categories of lipids which can be used to make the liposomes of this invention are straight chain fatty acids, glycerol esters, glycerides, phospho-glycerides, sphingolipids, waxes, terpenes and steroids. Specific preferred lipids suitable for use in this invention are phosphatidyl choline, dicetyl phosphate and cholesterol.

The melanin-liposome complex compositions of this invention are generally made by isolating a lipid mixture from its solvent, e.g. ethanol or chloroform; adding an aqueous solution of melanin in saline at about pH 8.8 to the evaporated lipid mixture and mixing to form a liposome-melanin complex. Optionally, a color modifier such as canthaxanthin can be included in the aqueous solution of melanin. The liposome-melanin complex is then recovered and combined with a suitable topical vehicle, e.g. a lotion or cream vehicle.

The lipid mixture which forms the liposome can be any of the conventional mixtures available or discussed in the literature which are pharmaceutically and cosmetically acceptable.

Preferred lipid mixtures contain a phosphatidyl choline, dicetyl phosphate and cholesterol. The lipid mixtures which form the liposomes are commercially available in a solvent such as ethanol or chloroform. A typical mixture contains on a weight basis, seven parts phosphatidylcholine, 2 parts dicetyl phosphate and one part cholesterol.

The melanin in the aqueous saline solution at a basic pH is preferably DOPA melanin in 0.9% sodium chloride at pH 8.8. A suitable color modifier in the aqueous solution can be canthaxanthin, however other color modifiers such as beta carotene and riboflavin or mixtures of the color modifiers can be used.

The concentrations of the melanin composition in the aqueous saline solution can vary depending on the ultimate color sought. However, a typical 5mL aqueous solution of 0.9% sodium chloride contains 0.01g DOPA melanin and 0.001g canthaxanthin.

The compositions of this invention demonstrated efficacy in lotions applied to SKH-1 hairless mice. The correlation between results in such mice and in humans relating to products which permeate the skin is discussed in Durrheim et al, Journal of Pharmaceutical Sciences, 69, No. 7, 781-786 (July 1980) and the similarity of effects of sunlight on hairless mice and humans is discussed by Poulsen et al, British Journal of Dermatology 110, 531-538 (1984).

Tests to determine the efficacy of the compositions were conducted as follows.

12 SKH-1 mice were separated into six groups of two each and identified as groups A, B, C, D, E and F. Topical lotions containing liposome - melanin complexes were applied dorsally to the mice in each of groups B, D and F. Each lotion contained $5 \times 10^{-4}$ g/mL melanin. A control topical lotion having the following composition was applied dorsally to the mice in each of groups A, C and E.

|  | Range |
| --- | --- |
| Part 1 |  |
| Lanolin | 0.2% - 1% |
| Cocoa Butter | 2.0% - 5% |
| Emcol RHT (Glyceryl Stearate)[1] | 2.0% - 4% |
| Hystrene 5016 (Stearic Acid)[2] | 2.0% - 4% |
| Vitamin E Acetate | 0.1% - 0.5% |
| Aloe Vera Lipo Quinone Extract | 0.1% - 1.0% |
| Jojoba Oil | 0.1% - 1.0% |
| Mineral Oil | 1.0% - 7% |
| Propylparaben | 0.1% - 1% |
| Medical Fluid 360 (Dimethicone)[3] | 0.1% - 1% |
| Part 2 |  |
| Water | 40% - 60% |
| Carbopol 941 (1%) (Polyacrylic Acid Polymer)[4] | 10% - 35% |
| Propylene Glycol | 2.0% - 7% |
| Triethanolamine 99% | 0.1% - 3% |
| Lanogel 41 (PEG-75 Lanolin)[5] | 0.25% - 1% |
| Methylparaben | 0.1% - 0.5% |
| Sequestrene Na$_2$ | 0.01% - 0.08% |
| Part 3 |  |
| Perfume | 0.01% - 0.5% |
| Heat parts 1 and 2 separately to 180° F. Add part 1 to part 2. Cool to 120° F and add part 3. Allow to cool to room temperature. | |

[1] Witco Corp., Organics Division, NY, NY (also Witconol RHT)
[2] Humko Chemical, Memphis, Tenn.
[3] Dow Corning Corp., Midland, Michigan
[4] B.F. Goodrich Specialty Polymers and Chemical Division, Cleveland, Ohio
[5] Amerchol Corp., Edison, NJ

Application density of the test and control lotions is 2 microliters/cm$^2$ with 15 minute drying time. The schedule of application is shown in Table I.

## TABLE I

| Group | #Applications/day | Day | Color |
|---|---|---|---|
| A (Control) | 1 | 1 | None |
| B (Test) | 1 | 1 | Light Beige |
| C (Control) | 1 | 2 | None |
| D (Test) | 1 | 2 | Light Beige |
| E (Control) | 2 | 2 | None |
| F (Test) | 2 | 2 | Tan |

At 24 hours post final application, the subjects are examined for development of coloration and sacrificed by cervical dislocation. The dorsal skin is stained for melanin by the Warthin-Starry cryostat method (Luna, L.H., Manual of Histologic Staining Methods of the Armed Forces Institute of Pathology, pp. 238-240, mcGraw-Hill Book Co., NY, NY (1968).

The results are as shown in the following Table II:

## TABLE II

| Group | Warthin Starry |
|---|---|
| A | no melanin detected; |
| B | good penetration of melanin into stratum corneum, small amount into epidermis |
| C | no melanin detected; |
| D | excellent penetration of melanin into stratum corneum, small amount into epidermis; |
| E | no melanin detected; |
| F | excellent penetration of melanin into stratum corneum, small amount into epidermis. |

The results in the tables show that melanin-liposome complexes impart color which resembles a suntan to the stratum corneum.

The following illustrate the preparation of the lotions used in the test.

## EXAMPLE 1

Control Lotion

Control lotion which was used on the mice in Groups A, C and E was prepared as follows:

5

|  | Range |
|---|---|
| Part 1 | |
| Lanolin | 0.2% - 1% |
| Cocoa Butter | 2.0% - 5% |
| Emcol RHT (Glyceryl Stearate)[1] | 2.0% - 4% |
| Hystrene 5016 (Stearic Acid)[2] | 2.0% - 4% |
| Vitamin E Acetate | 0.1% - 0.5% |
| Aloe Vera Lipo Quinone Extract | 0.1% - 1.0% |
| Jojoba Oil | 0.1% - 1.0% |
| Mineral Oil | 1.0% - 7% |
| Propylparaben | 0.1% - 1% |
| Medical Fluid 360 (Dimethicone)[3] | 0.1% - 1% |
| Part 2 | |
| Water | 40% - 60% |
| Carbopol 941 (1%) (Polyacrylic Acid Polymer)[4] | 10% - 35% |
| Propylene Glycol | 2.0% - 7% |
| Triethanolamine 99% | 0.1% - 3% |
| Lanogel 41 (PEG-75 Lanolin)[5] | 0.25% - 1% |
| Methylparaben | 0.1% - 0.5% |
| Sequestrene Na$_2$ | 0.01% - 0.08% |
| Part 3 | |
| Perfume | 0.01% - 0.5% |
| Heat parts 1 and 2 separately to 180° F. Add part 1 to part 2. Cool to 120° F and add part 3. Allow to cool to room temperature. | |

[1]Witco Corp., Organics Division, NY, NY (also Witconol RHT)
[2]Humko Chemical, Memphis, Tenn.
[3]Dow Corning Corp., Midland, Michigan
[4]B.F. Goodrich Specialty Polymers and Chemical Division, Cleveland, Ohio
[5]Amerchol Corp., Edison, NJ

EXAMPLE 2

Liposome-Melanin Complex

2 5ml containers, each having therein, 63 micromoles of L-alpha-phosphatidylcholine, 18 micromoles of dicetyl phosphate and 9 micromoles of cholesterol dissolved in 5 mL of chlorform were subjected to a rotary evaporator to evaporate the chloroform solvent.

A 5mL saline solution pH 8.7, with 20 drops (about 5mL) 0.5M NaOH, 0.01g dopa melanin and 0.0001g canthaxanthin was added into the evaporated lipids and mixed well. The resulting liposome-melanin complex was recovered.

EXAMPLE 3

Test Lotion

6

The test lotion which was used on the mice in Groups B, D and F was prepared as follows:

The liposome-melanin complex as prepared in Example 2 was mixed into the topical lotion as prepared in Example 1 to a concentration of 0.5 mL per gram of lotion.

Tests on humans were conducted to determine the effects of the lotion containing melanin-liposome complexes. The lotions used contained, in the melanin composition, 0.01g DOPA melanin and 0.0001 g canthaxanthin per 5mL of saline solution containing 20 drops of 0.5M NaOH.

## EXAMPLE 4

(a) The product of Example 3 was applied to excised mouse epidermis at 2 microliters per square centimeter. The color was excellent. The results of in vitro testing showed that the lotion provided protection against ultraviolet radiation.

(b) The product of Example 3 was applied to human skin. The color was excellent. Readings on a Minolta Chromameter showed that the color was in the natural tan range. After washing, Minolta Chromameter readings indicated that residual color remained in the stratum corneum.

## Claims

1. The method of sunless tanning of skin comprising applying to the skin a topical compositon containing from about $1 \times 10^{-6}$ mg/mL to about $5 \times 10^{-3}$ gm/mL of melanin, wherein the melanin is complexed with a liposome.

2. The method of claim 1 wherein the topical composition is a lotion.

3. The method of claim 1 wherein the melanin is DOPA melanin.

4. The method of claim 1, wherein the melanin is in a composition containing one or more color modifiers.

5. The method of claim 4, wherein the color modifier is selected from canthaxanthin, beta-carotene, riboflavin and a mixture thereof.

6. The method of claim 4, wherein the color modifier is a mixture of riboflavin and canthaxanthin.

7. The method of claim 4, wherein the color modifier is a mixture of canthaxanthin and beta-carotene.

8. A liposome composition comprising a melanin polymer composition entrapped in a liposome.

9. The composition of claim 8 wherein the melanin composition contains one or more color modifying agents.

10. The composition of claim 9, wherein the color modifier is selected from canthaxanthin, beta-carotene, riboflavin and a mixture thereof.

11. The composition of claim 9 wherein the color modifier is a mixture of riboflavin and canthaxanthin.

12. The composition of claim 9 wherein the color modifier is a mixture of canthaxanthin and beta-carotene.

13. The composition of claim 8 wherein the liposome comprises L-alpha-phosphatidylcholine, dicetyl phosphate and cholesterol.

14. The use of from about $1 \times 10^{-6}$ mg/mL to about $5 \times 10^{-3}$ gm/mL of melanin, wherein the melanin is complexed with a liposome, for the manufacture of a composition for the sunless tanning of skin.

15. The use of a composition comprising from about $1 \times 10^{-6}$ mg/mL to about $5 \times 10^{-3}$ gm/mL of melanin, wherein the melanin is complexed with a liposome, for the sunless tanning of skin.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 806 360  (LEONG et al.)<br>* Whole document *<br>--- | 1-12 | A 61 K   7/00<br>A 61 K   7/42 |
| A | US-A-4 806 344  (GASKIN)<br>* Whole document *<br>--- | 1-12 | |
| A | FR-A-2 597 367  (L'OREAL)<br>* Whole document *<br>--- | 1-15 | |
| P,A | JOURNAL OF PHOTOCHEMISTRY AND PHOTOBIOLOGY, vol. 3, no. 3, June 1989, pages 341-349, Elsecier Sequoia S.A., NL; A. EZZAHIR: "The influence of melanins on the photoperoxidation of lipids"<br>* Page 342, paragraph 2.1: "Chemicals" *<br>----- | 1-3 | |
| | | | **TECHNICAL FIELDS SEARCHED (Int. Cl.5)**<br><br>A 61 K |

The present search report has been drawn up for all claims

| Place of search | Date of completion of the search | Examiner |
|---|---|---|
| THE HAGUE | 16-05-1990 | FISCHER J.P. |